# EUROPEAN PATENT SPECIFICATION

(11) **EP 3 160 428 B1**
(45) Date of publication and mention of the grant of the patent: **07.08.2019**
(21) Application number: 15727678.3
(22) Date of filing: 11.06.2015
(51) Int. Cl.: A61Q 5/00, A61Q 5/02, A61Q 5/12, A61K 8/27, A61K 8/34, A61K 8/49, A61K 8/23

(54) **A SEBUM-PROMOTING AGENT FOR IMPROVING HAIR**
MITTEL ZUR FÖRDERUNG DER SEBUM-PRODUKTION ZUR VERBESSERUNG VON HAAR
AGENT PROMOTEUR DE LA PRODUCTION DE SÉBUM POUR AMÉLIORER LES CHEVEUX

(30) Priority: 24.06.2014 WO PCT/CN2014/080668; 29.08.2014 EP 14182855
(43) Date of publication of application: 03.05.2017
(73) Proprietor: Unilever NV, 3013 AL Rotterdam (NL); Unilever PLC, London EC4Y 0DY (GB)
(72) Inventor: BIAN, Xiaoying, Shanghai 200335 (CN); CAO, Qunhua, Shanghai 200335 (CN); VAN GORKOM, Leonardus, Cornelis, Maria, Trumbull, Connecticut 06611 (US); HOPTROFF, Michael John, Shanghai 200335 (CN); JAYASWAL, Amit, Bihar 811201 (IN); QIU, Jing, Shanghai 200335 (CN); ZHANG, Jing, Shanghai 200335 (CN)
(74) Representative: Chisem, Janet
(86) International application number: PCT/EP2015/063035
(87) International publication number: WO 2015/197370

(56) References cited:
- EP-A1- 0 348 015
- EP-A1- 1 504 752
- WO-A1-96/29983
- WO-A1-99/30675
- FR-A1- 2 943 255
- US-A1- 2011 143 984
- US-A1- 2012 145 175
- US-A1- 2014 060 567

## Description

### Field of the invention

The present invention relates to methods for improving hair by providing hair fibre benefits such as smoothness, strength, and breakage resistance. More particularly, the present invention relates to providing such benefits through stimulation or maintenance of sebum levels on scalp.

### Background of the invention

Many strategies have been developed for improving the condition of the hair. Most of these strategies involve applying products directly to the hair to coat the fibres with active materials and/or deliver active materials into the hair fibres. For example, conditioners and conditioning shampoos often contain silicone polymers which are deposited onto the hair fibres during rinsing. US 6,180,576 discloses one such conditioning shampoo composition that contains a dispersed, insoluble, nonionic silicone hair conditioning agent.

The present inventors have recognised a drawback with conventional strategies for improving hair in that the improvements may not always be achieved consistently and/or may persist for only a short time after applying product to the hair. Thus the present inventors have recognised a need to provide methods for improving hair wherein the hair fibre is conditioned naturally and preferably continually.

Sebum is naturally present on scalp but is often seen as a negative indicator of beauty and health through association with greasy hair or even a suggested association with more serious conditions such as seborrhoeic dermatitis. The present inventors have, however, surprisingly found that sebum itself can provide hair fibre benefits. In particular the inventors have found that the difference in sebum levels between dry and normal scalp is sufficient to translate into a significant difference in hair quality. Thus the inventors have recognized that interventions using agents that promote or maintain sebum on the scalp can be used to benefit hair fibres.

The use of sebum promoting agents for moisturizing and softening skin has been described in WO 96/29983 A. However no suggestion is made in WO 96/29983 A that such agents may provide a benefit for hair fibres. US2012/145175 discloses a hair transformation agent, useful in transforming hair texture from kinky to curly by application of a non-permanent solution which acts like the sebum of a human. The drawback in this method is that generous portions of the composition need to be applied and left on to the hair to provide the desired benefit.

### Summary of the invention

The present invention provides use of a metal pyrithione as sebum-promoting agent or a composition comprising the agent for providing a hair fibre benefit selected from one or more of smoothness, strength, and breakage resistance.

The invention may also be described as a method of providing a hair fibre benefit selected from one or more of smoothness, strength and breakage resistance, the method comprising applying metal pyrithione as sebum-promoting agent or a composition comprising metal pyrithione to the scalp of an individual in need of said hair benefit.

The invention may also be described as a method of directing an individual to apply metal pyrithione as sebum-promoting agent to their scalp in order to receive a hair fibre benefit selected from one or more smoothness, strength and breakage resistance.

All other aspects of the present invention will more readily become apparent upon considering the detailed description and examples which follow.

### Detailed description

Sebum-promoting agents are substances which, when applied topically to the scalp, promote the natural production of sebum by the skin. The term "promote the natural production of sebum" as used herein encompasses restoring sebum levels, increasing sebum levels and/or maintaining sebum levels. The agent may be used in substantially pure form or as part of a composition. Sebum is mainly composed of lipids and so the sebum may additionally or alternatively be termed as "natural scalp lipids". Similarly the agent may additionally or alternatively be termed as a "natural lipid promoting agent".

Sebum-promoting agents are described, for example, in WO 96/29983 A, (see page 17 (line 35) to page 19 (line 2)). The sebum-promoting agent of the present invention is metal pyrithione, especially zinc pyrithione.

By the term "hair fibre benefit", and related terms used herein, we mean improvement and/or maintenance of the quality of the hair fibre itself rather than the number or density of growth of such fibres. Examples of fibre benefit include, for example, one or more of: smoothness, shine, sheen, silkiness, strength, breakage resistance, damage resistance, and damage repair. The fibre benefits addressed in the present invention are smoothness, strength, breakage resistance and damage resistance. Alternative descriptions for hair strength, which are encompassed by the term as used herein, include, but are not limited to, one or more of the following: resilience, durability, vigour, vitality, and healthiness.

The sebum-promoting agent is preferably applied to the scalp such that the scalp sebum level is restored or maintained in an amount sufficient to provide the hair fibre benefit and avoid dryness of the scalp. In particular it is preferred that the level of scalp sebum is at least 30 µg / cm², more preferably at least 35 µg / cm², and most preferably at least 40 µg / cm².

The sebum-promoting agent is preferably applied to the scalp such that the scalp sebum level is restored or maintained in an amount sufficient to provide the hair fibre benefit without imparting greasiness. In particular it is preferred that the level of scalp sebum is no greater than 120 µg / cm², more preferably no greater than 100 µg / cm², more preferably still no greater than 80 µg / cm², and most preferably no greater than 60 µg / cm².

Sebum levels referred to herein means those determined using a Sebumeter and preferably are measured using a Sebumeter SM 815® (Courage & Khazaka electronic GmbH). Measurement should be taken along a hair parting at visibly dry regions on the scalp, if present. Preferably measurements are taken 48 hours after hair washing as described in Example 2 herein below.

The sebum-promoting agent may be used in substantially pure form. However in a preferred embodiment the agent is used in the invention in the form of a hair treatment composition, such as a shampoo, conditioner or leave-on treatment. Typically, the agent is present in the composition in an amount of from 0.001 to 5%, more preferably 0.01 to 2%, more preferably still 0.05 to 1%, and most preferably 0.1 to 0.5% by weight of the composition.

Preferably, the composition comprises at least 5% of water by weight of the composition, more preferably from 15 to 95%, even more preferably from 35 to 88%, still even more preferably from 45 to 82%, most preferably from 65 to 80% by weight of the total composition.

Compositions of the invention are primarily intended for topical application to at least a portion of the scalp (and optionally the hair) of an individual, either as rinse-off or leave-on compositions.

Most preferred are rinse-off compositions, especially shampoos and conditioners. The composition for enabling the use as per the present invention is preferably a rinse-off composition because rinse-off compositions like shampoos and conditioners not only provide the cleaning and/or conditioning benefits but leave only the necessary amount of a sebum promoting agent (which are generally non-oily in nature) deposited on the scalp to enable secretion of the right level of natural sebum to provide the hair fibre benefit of the present invention. Such a benefit when provided for through a leave-on composition may be less preferred by the consumer due to the sticky and/ or oily feel given by oils used to deliver the sebum-promoting agents when directly applied on the hair/ scalp and left on there to deliver the benefit.

Shampoo compositions according to the invention will generally comprise one or more anionic cleansing surfactants which are cosmetically acceptable and suitable for topical application to the hair.

Examples of suitable anionic cleansing surfactants are the alkyl sulphates, alkyl ether sulphates, alkaryl sulphonates, alkanoyl isethionates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof, especially their sodium, magnesium, ammonium and mono-, di- and triethanolamine salts. The alkyl and acyl groups generally contain from 8 to 18, preferably from 10 to 16 carbon atoms and may be unsaturated. The alkyl ether sulphates, alkyl ether sulphosuccinates, alkyl ether phosphates and alkyl ether carboxylic acids and salts thereof may contain from 1 to 20 ethylene oxide or propylene oxide units per molecule.

Preferred anionic cleansing surfactants for use in shampoo compositions of the invention include sodium oleyl succinate, ammonium lauryl sulphosuccinate, sodium lauryl sulphate, sodium lauryl ether sulphate, sodium lauryl ether sulphosuccinate, ammonium lauryl sulphate, ammonium lauryl ether sulphate, sodium dodecylbenzene sulphonate, triethanolamine dodecylbenzene sulphonate, sodium cocoyl isethionate, sodium lauryl isethionate, lauryl ether carboxylic acid and sodium N-lauryl sarcosinate. Most preferred anionic cleansing surfactants are selected from sodium lauryl sulphate, sodium lauryl ether sulphate (n)EO, (where n is from 1 to 3), sodium lauryl ether sulphosuccinate(n)EO, (where n is from 1 to 3), ammonium lauryl sulphate, ammonium lauryl ether sulphate(n)EO, (where n is from 1 to 3), sodium cocoyl isethionate, lauryl ether carboxylic acid (n) EO (where n is from 10 to 20), and mixtures thereof.

Mixtures of any of the foregoing anionic cleansing surfactants may also be suitable.

The total amount of anionic cleansing surfactant in shampoo compositions of the invention generally ranges from 0.5 to 30%, more preferably from 1.5 to 25%, and most preferably from 5 to 18% by weight of the composition.

Conditioner compositions according to the invention may typically comprise one or more cationic conditioning surfactants which are cosmetically acceptable and suitable for topical application to the hair.

Preferably, the cationic conditioning surfactants have the formula N⁺(R¹)(R²)(R³)(R⁴), wherein R¹, R², R³ and R⁴ are independently (C₁ to C₃₀) alkyl or benzyl.

Preferably, one, two or three of R¹, R², R³ and R⁴ are independently (C₄ to C₃₀) alkyl and the other R¹, R², R³ and R⁴ group or groups are (C₁-C₆) alkyl or benzyl.

More preferably, one or two of R¹, R², R³ and R⁴ are independently (C₆ to C₃₀) alkyl and the other R¹, R², R³ and R⁴ groups are (C₁-C₆) alkyl or benzyl groups. Optionally, the alkyl groups may comprise one or more ester (-OCO- or -COO-) and/or ether (-O-) linkages within the alkyl chain. Alkyl groups may optionally be substituted with one or more hydroxyl groups. Alkyl groups may be straight chain or branched and, for alkyl groups having 3 or more carbon atoms, cyclic. The alkyl groups may be saturated or may contain one or more carbon-carbon double bonds (e.g., oleyl). Alkyl groups are optionally ethoxylated on the alkyl chain with one or more ethyleneoxy groups.

Suitable cationic surfactants include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, dihydrogenated tallow dimethyl ammonium chloride, cocotrimethylammonium chloride, PEG-2-oleammonium chloride and the corresponding hydroxides thereof. Further suitable cationic surfactants include those materials having the CTFA designations Quaternium-5, Quaternium-31 and Quaternium-18. Mixtures of any of the foregoing materials may also be suitable. Particularly useful cationic surfactant for use in conditioners according to the invention is cetyltrimethylammonium chloride, behenyltrimethylammonium chloride or a mixture thereof.

Another example of a class of suitable cationic conditioning surfactants for use in the invention, either alone or in admixture with one or more other cationic conditioning surfactants, is a combination of (i) and (ii) below:
(i) an amidoamine corresponding to the general formula (I): 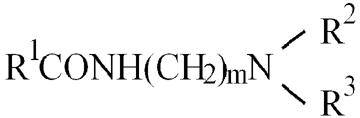
   in which R¹ is a hydrocarbyl chain having 10 or more carbon atoms,
   R² and R³ are independently selected from hydrocarbyl chains of from 1 to 10 carbon atoms, and
   m is an integer from 1 to about 10; and
(ii) an acid.

As used herein, the term hydrocarbyl chain means an alkyl or alkenyl chain.

Preferred amidoamine compounds are those corresponding to formula (I) in which
R¹ is a hydrocarbyl residue having from about 11 to about 24 carbon atoms,
R² and R³ are each independently hydrocarbyl residues, preferably alkyl groups, having from 1 to about 4 carbon atoms, and m is an integer from 1 to about 4.

Preferably, R² and R³ are methyl or ethyl groups.

Preferably, m is 2 or 3, i.e. an ethylene or propylene group.

Preferred amidoamines useful herein include stearamido-propyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethylamine, behenamidopropyldiethylmine, behenamidoethyldiethylamine, behenamidoethyldimethylamine, arachidamidopropyldimethylamine, arachidamidopropyldiethylamine, arachid-amidoethyldiethylamine, arachidamidoethyldimethylamine, and mixtures thereof.

Particularly preferred amidoamines useful herein are stearamidopropyldimethylamine, stearamidoethyldiethylamine, and mixtures thereof.

Acid (ii) may be any organic or mineral acid which is capable of protonating the amidoamine in the hair treatment composition. Suitable acids useful herein include hydrochloric acid, acetic acid, tartaric acid, fumaric acid, lactic acid, malic acid, succinic acid, and mixtures thereof. Preferably, the acid is selected from the group consisting of acetic acid, tartaric acid, hydrochloric acid, fumaric acid, lactic acid and mixtures thereof.

The primary role of the acid is to protonate the amidoamine in the conditioner composition thus forming a tertiary amine salt (TAS) in situ in the composition. The TAS in effect is a non-permanent quaternary ammonium or pseudo-quaternary ammonium cationic surfactant.

Suitably, the acid is included in a sufficient amount to protonate all the amidoamine present, i.e. at a level which is at least equimolar to the amount of amidoamine present in the composition.

In conditioners of the invention, the level of cationic conditioning surfactant will generally range from 0.01 to 10%, more preferably 0.05 to 7.5%, most preferably 0.1 to 5% by weight of the composition.

Conditioners of the invention will typically also incorporate a fatty alcohol.

Representative fatty alcohols comprise from 8 to 22 carbon atoms, more preferably 16 to 22. Fatty alcohols are typically compounds containing straight chain alkyl groups. Examples of suitable fatty alcohols include cetyl alcohol, stearyl alcohol and mixtures thereof.

The level of fatty alcohol in conditioners of the invention will generally range from 0.01 to 10%, preferably from 0.1 to 8%, more preferably from 0.2 to 7%, most preferably from 0.3 to 6% by weight of the composition.

The sebum-promoting agent or composition comprising the agent is preferably applied to the scalp of the individual at least twice per week, more preferably at least three times per week. In one embodiment the sebum-promoting agent or composition comprising the agent is preferably applied to the scalp of the individual three times per week. In another embodiment the sebum-promoting agent or composition comprising the agent is preferably applied to the scalp of the individual once or twice per day. In one embodiment, the individual to which the agent or composition is applied may exhibit dry scalp and/or be in need of scalp moisturization. Additionally or alternatively the individual may have a scalp sebum level of less than 30 µg / cm² prior to application.

The sebum-promoting agent or composition comprising the agent is preferably applied to the scalp of the individual in each application in an amount of from 0.0001 to 0.2 g in terms sebum-promoting agent (e.g. if a shampoo composition contains 1 wt% agent and is applied in an amount of 2 g shampoo then the amount applied in terms of sebum-promoting agent is 0.02 g). More preferably the sebum-promoting agent or composition comprising the agent is applied to the scalp of the individual in an amount of from 0.001 to 0.1 g in terms sebum-promoting agent, even more preferably from 0.002 to 0.07 g and most preferably from 0.006 to 0.05 g.

Each application may be from the same composition or product format (e.g. each application may be from a shampoo composition). Alternatively, different compositions and/or product formats may be used (e.g. the agent may be applied in a shampoo composition and subsequently in a conditioner composition).

The agent and/or composition is preferably associated with indicia directing an individual to apply the same to the individual's scalp in order to receive the hair fibre benefit. The indicia may be in the form of text, logo and/or graphics and may be communicated through various media including one or more of, product label, printed advertising material (including pamphlets, posters, magazines, newspapers or a combination thereof), visual display units (including screens of televisions, monitors, portable digital device screens and combinations thereof), and speaker systems (including in-store address systems, speaker systems of televisions, radios, computers and/or portable digital devices).

Except in the examples, or where otherwise explicitly indicated, all numbers in this description indicating amounts of material or conditions of reaction, physical properties of materials and/or use may optionally be understood as modified by the word "about".

All amounts are by weight of the composition, unless otherwise specified.

It should be noted that in specifying any range of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

The following examples are provided to facilitate an understanding of the invention. The examples are not intended to limit the scope of the claims.

### Examples

### Example 1

Sample shampoo and conditioner formulations suitable for use in the present invention are shown in Tables 1 and 2.

**TABLE 1**

| **Ingredient % wt.** | **Shampoo 1** | **Shampoo 2** | **Shampoo 3** |
|---|---|---|---|
| Sodium Laureth Sulphate | 16 | 14 | 14 |
| Cocoamidopropyl betaine | 2 | 1.6 | 1.6 |
| Zinc Pyrithione | 0.25 | 1.0 | 0.25 |
| Zinc Sulfate | --- | 0.1 | 0.1 |
| Zinc Carbonate | 0.25 | --- | --- |
| Silicone | 3.0 | 2.0 | 2.0 |
| Climbazole | --- | 0.5 | --- |
| Acrylic Acid Polymer (Carbomer) | --- | 0.6 | 0.6 |
| Ethyleneglycol Distearate (EGDS) | 1.5 | --- | |
| Guar Hydroxypropyl Trimonium Chloride | 0.4 | 0.2 | 0.2 |
| Salt, Preservatives and Perfumes | 1.0 | 1.5 | 1.5 |
| Water | To 100 | To 100 | To 100 |

**TABLE 2**

| **Ingredient % wt.** | **Conditioner** |
|---|---|
| Behenyltrimmonium chloride | 0.875 |
| Stearamidopropyl dimethylamine | 1.25 |
| Cetearyl alcohol | 5.00 |
| Silicone | 3.00 |
| Zinc pyrithione | 0.25 |
| Zinc sulphate | 0.1 |
| Sodium chloride | 0.1 |
| Perfume | 0.6 |
| Thickener | 0.20 |
| Lactic acid | 0.38 |
| Preservatives | 0.31 |
| Water | to 100 |

### Example 2

This example demonstrates the effect of applying shampoo and conditioner comprising zinc pyrithione (ZnPTO) on sebum levels in dry scalp.

A clinical study was conducted to compare the ability of a 0.25 wt% ZnPTO shampoo and 0.25 wt% ZnPTO conditioner system with that of a marketed beauty shampoo and conditioner system (free from sebum-promoting agents) to elicit an increase in casual scalp sebum level over a period of regular test product use.

A half head, double-blind, randomised design was employed. A one week run-in phase using a standard (i.e. free-from sebum-promoting agent) shampoo at home, was followed by several weeks of half head salon washing 3 times per week. The two treatment systems were randomly allocated to the left and right side of each head.

A total of 125 female subjects with ages ranging from 18 to 60 and of various ethnicities participated in the trial. All participants had areas of visibly dry scalp but were otherwise free from scalp disorders. Scalp assessment was performed using ASFS as reported in "Assessing therapeutic effectiveness of scalp treatments for dandruff and seborrheic dermatitis, part 1: a reliable and relevant method based on the adherent scalp flaking score (ASFS)", Journal of Dermatological Treatment, 2014, 25(3), 232-6. Subjects were included if the assessment revealed slight or mild dryness on both sides of scalp at screening. Specifically, the inclusion criteria were: subject must have no score of 6 or above even if below 40% of octant; subject must have slight or mild dryness in at least 2 octants on each side of head covering at least 10% of the octant; subject must not have total ASFS score of 24 or greater.

Clinical evaluations of casual surface sebum were conducted at baseline and then after regular intervals during the weeks of treatment. All assessments were carried out 48 ± 4 hours after a wash.

Sebum measurements were made as follows: A Sebumeter SM 815® (Courage & Khazaka electronic GmbH) was used. Subjects were required to sit calmly in a temperature and humidity controlled room (temperature range 20 ± 2 °C, relative humidity range 30-40 %) with the partings in place for approximately 15 minutes prior to all measurements. Measurements were taken at a visually dry site and a visually healthy site per half head. Two measurements were taken side by side on the parting at each of the specified sites at baseline. The locations of the sites were recorded and the same sites measured at each time point throughout the study. The mean of the replicates for each site were calculated.

For scalp sebum, average scores at visually dry sites were significantly higher for the 0.25% ZnPTO system than for the beauty system for all time points from wash 9 (week 3) through to wash 21 (week 7) (p<0.05). The baseline mean sebum level (with 95% confidence interval in parentheses) at visually dry sites was 25 (21 - 29) µg / cm². For the 0.25% ZnPTO system this increased to 35 (31 - 41)□ µg / cm² at wash 9.

For visually healthy sites, mean sebum levels were greater than 35□µg / cm² at all time points and never exceeded 80 µg / cm².

### Example 3

This example demonstrates the effect of sebum levels on hair smoothness.

Hair switches were treated with model sebum having the composition given in Table 3.

**TABLE 3**

| **Ingredient** | **% w/w** |
|---|---|
| Oleic acid | 8 |
| Isostearic acid | 4 |
| Tricaprin | 1.8 |
| Triolein (65%) | 28.2 |
| Triisostearin | 9.2 |
| oleyl oleate | 11.9 |
| myristyl myristate | 11.9 |
| isostearyl isostearate | 6 |
| squalene | 13.8 |
| cholesterol oleate | 3.4 |
| cholesterol | 1.7 |

The data from the study in Example 2 were used to calculate the amount of model sebum applied to the switches. This calculation involved assumptions including that the sebumeter detects 40% of total skin surface lipids, hair density on scalp is 272.97 hair / cm² and 80% of sebum on scalp is transferred to hair. Using these assumptions and the average number of hairs in a switch of long hair resulted in a determination that 0.705 µl of model sebum per 2.5 g switch and 0.987 µl model sebum per switch were required to simulate hair in subjects with baseline (25□µg / cm²) and treated (35□µg / cm²) scalp sebum levels respectively.

The model sebum was applied to the switches at the root and spread along the switch by combing the switch from root to tip. Each switch was then stored hung vertically from the root for a period of 20 hours prior to measurement.

Texture analysis of the hair switches was then performed using a TA.XT plus Texture Analyser. The texture analysis revealed that the switches treated with 0.987 µl model sebum had a significantly lower friction (P<0.05) than those treated with 0.705 µl of model sebum.

### Example 4

This example demonstrates the effect of sebum levels on hair strength.

Switches were prepared in an identical manner to that described in Example 3 except the model sebum was applied to each 2.5 g switch by soaking in 10 ml of a mixture of the model sebum and hexane followed by drying at room temperature (25 °C) in a fume hood for 2 hours. Each switch was then stored hung vertically from the root for a period of 20 hours prior to measurement.

Micro tensile analysis of the hair switches was then performed using a Tensile Tester Type MTT-680. The tensile tests revealed that the switches treated with 0.987 µl model sebum had a significantly higher (P<0.05) elastic modulus than those treated with 0.705 µl of model sebum.

### Example 5

This example demonstrates the effect of sebum levels on damage resistance.

Required model sebum levels were calculated in a similar manner as in Example 3 except that an additional assumption was made that sebum initially transfers from the scalp to 1 mm of hair root, resulting in a determination that 12.6 µl of model sebum per 0.8 g switch (representing the 1 mm of hair closest to the root across an entire scalp) and 17.65 µl model sebum per 0.8 g switch were required to simulate hair at the roots of subjects with baseline (25□µg / cm²) and treated (35□µg / cm²) scalp sebum levels respectively.

The sebum was applied to the switches which were then stored hung vertically from the root for a period of 20 hours prior to measurement.

Differential scanning calorimetry (DSC) of the hair switches was then performed. The DSC analysis revealed that the switches treated with 17.65 µl artificial sebum had a significantly higher (P<0.05) denaturation temperature than those treated with 12.6 µl of artificial sebum.

### Example 6

This example demonstrates the natural penetration of sebum lipids into hair fibre.

Raman spectroscopy was used to anaylse the squalene levels across a hair fibre cross-section. Briefly, microtomed hair sections were examined using a Horiba, HR Evolution spectrometer with a 785 nm laser source and a grating size of 600 gr/mm (spectral resolution of 2 cm⁻¹). The scanning range was between 450 cm⁻¹ and 1750 cm⁻¹, and the spectrum can be acquired with two windows. Each spectrum was collected with a total of 100 seconds laser exposure (50 seconds for each window). By collecting 4 spectra from single spot and taking an average of these, it was possible to obtain a good Raman spectrum on hair with reasonable signal-to-noise ratio (S/N). The cosmic rays were removed. The spectrum of squalene was obtained by using same parameters, with a collection time of 5 seconds to avoid signal saturation. Two dimensional (2D) maps on hair section were collected by scanning multiple points with same parameters and a step size of 3 µm was used on x-y plane.

All the spectra were processed (baseline correction) by instrument built-in functions, and analysed by using custom-written Matlab program for multi-variate curve resolution (MCR) analysis.

The results showed that squalene was present within the hair fibre in a ring-shaped and discrete distribution. Without wishing to be bound by theory, we postulate that the squalene penetrates the hair fibre as part of the scalp-derived sebum and its presence within the hair fibre contributes to at least some of the fibre benefits.

## Claims

1. Use of a sebum-promoting agent or a composition comprising the agent for providing a hair fibre benefit wherein the sebum-promoting agent comprises metal pyrithione; and wherein the hair fibre benefit is selected from one or more of smoothness, strength, and breakage resistance.

2. Use as claimed in any one of the preceding claims wherein the sebum-promoting agent or the composition comprising the agent restores or maintains scalp sebum in an amount sufficient to provide the hair fibre benefit without imparting greasiness.

3. Use as claimed in claim 2 wherein the scalp sebum is restored or maintained in an amount of from 30 to 120 µg / cm², preferably 35 to 100 µg / cm².

4. Use as claimed in any one of the preceding claims wherein the composition comprises the sebum-promoting agent in an amount of from 0.05 to 1% by weight of the composition, preferably 0.1 to 0.5%.

5. Use as claimed in any one of the preceding claims wherein the composition is a rinse-off composition.

6. Use as claimed in claim 5 wherein the composition is selected from shampoo and conditioner.

7. Use as claimed in any one of the preceding claims wherein the sebum-promoting agent or composition comprising the agent is applied to the scalp of the individual at least twice per week.

8. Use as claimed in claim 7 wherein the sebum-promoting agent or composition comprising the agent is applied to the scalp of the individual at least three times per week.

9. Use as claimed in any one of the preceding claims wherein the sebum-promoting agent or composition comprising the agent is applied to the scalp of the individual in an amount of from 0.0001 to 0.2 g of the sebum-promoting agent.

## Patentansprüche

1. Verwendung eines Sebum-fördernden Mittels oder einer Zusammensetzung, die das Mittel zum Erzielen eines Haarfaservorteils umfasst, wobei das Sebum-fördernde Mittel Metallpyrithion umfasst und wobei der Haarfaservorteil unter einer oder mehreren von Glätte, Festigkeit und Bruchbeständigkeit ausgewählt ist.

2. Verwendung, wie in irgendeinem der vorhergehenden Ansprüche beansprucht, wobei das Sebum-fördernde Mittel oder die Zusammensetzung, die das Mittel umfasst, Kopfhautsebum in einer ausreichenden Menge wiederherstellt oder aufrecht erhält, um den Haarfaservorteil zu erzielen, ohne dass sich Fettigkeit einstellt.

3. Verwendung, wie im Anspruch 2 beansprucht, wobei das Kopfhautsebum in einer Menge von 30 bis 120 µg/cm², vorzugsweise von 35 bis 100 µg/cm², wiederhergestellt oder aufrechterhalten wird.

4. Verwendung, wie in irgendeinem der vorhergehenden Ansprüche beansprucht, wobei die Zusammensetzung das Sebum-fördernde Mittel in einer Menge von 0,05 bis 1 Gewichts-% der Zusammensetzung, vorzugsweise von 0,1 bis 0,5 Gewichts-%, umfasst.

5. Verwendung, wie in irgendeinem der vorhergehenden Ansprüche beansprucht, wobei die Zusammensetzung eine Abspülzusammensetzung darstellt.

6. Verwendung, wie im Anspruch 5 beansprucht, wobei die Zusammensetzung unter Shampoo und Konditioniermittel ausgewählt ist.

7. Verwendung, wie in irgendeinem der vorhergehenden Ansprüche beansprucht, wobei das Sebum-fördernde Mittel oder die Zusammensetzung, die das Mittel umfasst, mindestens zweimal pro Woche auf die Kopfhaut des Individuums aufgetragen wird.

8. Verwendung, wie im Anspruch 7 beansprucht, wobei das Sebum-fördernde Mittel oder die Zusammensetzung, die das Mittel umfasst, mindestens dreimal pro Woche auf die Kopfhaut des Individuums aufgetragen wird.

9. Verwendung, wie in irgendeinem der vorhergehenden Ansprüche beansprucht, wobei das Sebum-fördernde Mittel oder die Zusammensetzung, die das Mittel umfasst, in einer Menge von 0,0001 bis 0,2 g Sebum-förderndes Mittel auf die Kopfhaut des Individuums aufgetragen wird.

## Revendications

1. Utilisation d'un agent de promotion de sébum ou d'une composition comprenant l'agent pour fournir un bénéfice à la fibre capillaire dans laquelle l'agent de promotion de sébum comprend une pyrithione de métal ; et dans laquelle le bénéfice à la fibre capillaire est choisi parmi un ou plusieurs de lissé, résistance et résistance à la rupture.

2. Utilisation selon l'une quelconque des revendications précédentes, dans laquelle l'agent de promotion de sébum ou la composition comprenant l'agent restaure ou maintient le sébum du cuir chevelu dans une quantité suffisante pour fournir le bénéfice à la fibre capillaire sans communiquer de graissage.

3. Utilisation selon la revendication 2, dans laquelle le sébum de cuir chevelu est restauré ou maintenu dans une quantité de 30 à 120 µg/cm², de préférence de 35 à 100 µg/cm².

4. Utilisation selon l'une quelconque des revendications précédentes, dans laquelle la composition comprend l'agent de promotion de sébum dans une quantité de 0,05 à 1 % en masse de la composition, de préférence de 0,1 à 0,5 %.

5. Utilisation selon l'une quelconque des revendications précédentes, dans laquelle la composition est une composition sans rinçage.

6. Utilisation selon la revendication 5, dans laquelle la composition est choisie parmi un shampoing et un après-shampoing.

7. Utilisation selon l'une quelconque des revendications précédentes, dans laquelle l'agent de promotion de sébum ou la composition comprenant l'agent est appliqué au cuir chevelu de l'individu au moins deux fois par semaine.

8. Utilisation selon la revendication 7, dans laquelle l'agent de promotion de sébum ou la composition comprenant l'agent est appliqué au cuir chevelu de l'individu au moins trois fois par semaine.

9. Utilisation selon l'une quelconque des revendications précédentes, dans laquelle l'agent de promotion de sébum ou la composition comprenant l'agent est appliqué au cuir chevelu de l'individu dans une quantité de 0,0001 à 0,2 g de l'agent de promotion de sébum.
